# FASCICULE DE BREVET EUROPEEN

(11) **EP 3 812 745 B1**
(45) Date de publication et mention de la délivrance du brevet: **05.07.2023**
(21) Numéro de dépôt: 20201570.7
(22) Date de dépôt: 13.10.2020
(51) Int. Cl.: G01N 21/64, G01N 33/22, G01N 33/28, B01L 3/00

(54) **DISPOSITIF DE MESURE DE LA STABILITE D'UN CARBURANT AU MOYEN DE MESURES DE VARIATION DE LA CAPACITE THERMIQUE ET DE LA FLUORESCENCE**
VORRICHTUNG ZUR MESSUNG DER STABILITÄT EINES BRENNSTOFFS MITHILFE VON MESSUNGEN DER ÄNDERUNG DER THERMISCHEN KAPAZITÄT UND FLUORESZENZ
DEVICE FOR MEASURING THE STABILITY OF A FUEL BY MEANS OF MEASUREMENTS OF VARIATION IN THERMAL CAPACITY AND FLUORESCENCE

(30) Priorité: 21.10.2019 FR 1911765
(43) Date de publication de la demande: 28.04.2021
(73) Titulaire: IFP Energies nouvelles, 92852 Rueil-Malmaison Cedex (FR)
(72) Inventeur: BORDES, Emilie, 92852 RUEIL-MALMAISON CEDEX (FR); DALMAZZONE, Christine, 92852 RUEIL-MALMAISON CEDEX (FR); ALVES FORTUNATO, Maira, 92852 RUEIL-MALMAISON CEDEX (FR)
(74) Mandataire: IFP Energies nouvelles

(56) Documents cités:
- FR-A1- 3 071 062
- US-A1- 2007 187 617
- Michal Borecki ET AL: "Capillary Sensor with UV-VIS Reading of Effects of Diesel and Biodiesel Fuel Degradation in Storage", Sensors & Transducers, octobre 2016 (2016-10), pages 1-9, XP055474884, Extrait de l'Internet: URL:http://www.sensorsportal.com/HTML/DIGE ST/october_2016/Vol_205/P_2861.pdf [extrait le 2020-06-19]

## Description

### Domaine technique

La présente invention concerne le domaine de l'analyse et de la caractérisation d'un carburant, notamment d'un carburant de type Diesel, biodiesel, essence ou carburéacteur (carburant utilisé en aéronautique également appelé jet fuel mais aussi kérosène par abus de langage).

Elle concerne plus particulièrement un dispositif de caractérisation permettant la mesure de la stabilité à l'oxydation et/ou la stabilité thermique du carburant.

L'émergence de nouvelles filières de production des carburants et biocarburants a introduit une variabilité importante de la formulation des carburants terrestres et aéronautiques sur le marché mondial. Les propriétés physico-chimiques macroscopiques (densité, viscosité, etc.) des nouvelles formulations sont semblables à celles des carburants fossiles traditionnels afin de permettre leurs utilisations dans les systèmes moteurs actuels (carburants dit « drop-in »). Cependant, les molécules, les familles chimiques présentes, et les impuretés ne sont pas identiques. Cela a un impact sur plusieurs propriétés d'usage des carburants comme la stabilité thermo-oxydative, le pouvoir lubrifiant, la corrosion, entre autres.

La dégradation de ces carburants par des processus d'oxydation peut entrainer la formation de produits insolubles et de dépôts d'origines et de compositions diverses dans les réservoirs de carburant, les circuits carburants, les systèmes de combustion et les circuits d'injection. Ces dépôts peuvent être à l'origine de dysfonctionnements mécaniques des pompes et des injecteurs mais aussi de colmatages de filtre et donc conduire à un accroissement des pertes de charges. La dégradation des biocarburants et carburants alternatifs dans le domaine des transports terrestres concerne tant les biocarburants de type esters méthyliques d'acide gras (EMAG) que ceux de type huiles végétales hydrotraitées (HVO). Dans le cas des carburéacteurs pour l'aéronautique, la stabilité à l'oxydation concerne toutes les filières (par exemple les procédés basés sur la thermochimie comme HEFA-SPK, Esters d'acides gras hydrotraités - Kérosène paraffinique ou ceux basés sur la biotechnologie, comme ATJ-SPK (Alcool vers Jet - Kérosène paraffinique synthétique).

De plus, dans le domaine automobile, les normes anti-pollution imposées aux véhicules incitent les industriels à développer des moteurs de plus en plus performants afin de réduire les émissions à la source, c'est-à-dire, dès la combustion, ce qui se traduit par une technologie actuelle des moteurs à combustion avec des conditions opératoires des systèmes d'injection diesel de plus en plus sévères, avec une augmentation des contraintes thermiques (T>150'), une augmentation de la pressio n (P>2500 bar) associées à une diminution du diamètre des trous des injecteurs.

Dans le domaine aéronautique, les cycles de montée en température et en pression imposés au carburant associés à des conditions qui se sévèrisent sont critiques pour maintenir un produit stable.

Dans le domaine logistique, il apparaît que la chaîne logistique devient de plus en plus complexe avec de nombreux produits différents associés à des additifs variés et nouveaux.

La stabilité des carburants suscite par conséquent un intérêt considérable de la part des acteurs du domaine aéronautique et du domaine des véhicules terrestres mais aussi du domaine du raffinage.

### Technique antérieure

Ces différents points poussent à une caractérisation de la stabilité thermique du carburant très détaillée afin d'éviter des problèmes causés par la dégradation des carburants dans l'automobile, l'aéronautique et le raffinage logistique (ex. : dépôts). Actuellement, il y a une demande croissante des industriels du secteur de l'automobile (constructeurs automobiles, équipementiers, pétroliers, etc.), notamment en Europe, pour une caractérisation de la stabilité thermique de manière plus représentative des nouvelles contraintes subies par les carburants.

Aujourd'hui, il n'y a pas de technique certifiée pour étudier la perte de stabilité thermique et la formation de dépôts des carburants diesel en condition dynamique. Cela est lié à la complexité des différentes configurations moteurs ainsi qu'à la difficulté de trouver un test représentatif. Les caractérisations des carburants diesel sont plutôt axées sur la stabilité à l'oxydation avec par exemple les méthodes PetroOxy (selon la norme ASTM 7545) et Rancimat (selon la norme EN15751). Dans le cas de l'industrie aéronautique, la stabilité thermique des carburéacteurs est contrôlée et mesurée à travers le test JFTOT ^{™} (Jet Fuel Thermal Oxidation Tester, pour test d'oxydation thermique des carburéacteurs). Un exemple de cette méthode est décrit dans le brevet US5293218 A et dans la méthode standard ASTM D3241-14be1. Le test JFTOT ^{™} consiste à faire circuler un carburéacteur autour d'un tube chauffé. Cependant, un test du type JFTOT ^{™} dédié au carburant diesel est actuellement inexistant dans la norme et dans la spécification carburant. De plus, cet équipement présente les inconvénients d'être encombrant, d'avoir une interprétation difficile. Il peut être par le choix des matériaux. En outre, le test de type JFTOT ^{™} nécessite une quantité de carburant importante et un environnement sécurisé.

Afin de réduire l'encombrement, certains moyens de test de carburant utilisent des puces micro-fluidiques. Un exemple d'un tel moyen de test d'oxydation de carburant est décrit dans la demande de brevet BR PI 1002057-8 A2. Le test spécifique décrit dans ce document concerne une oxydation accélérée au moyen d'injection d'ozone. Toutefois, le dispositif décrit dans cette demande de brevet est complexe, car il nécessite l'injection d'ozone dans le carburant. En outre, la cellule micro-fluidique qui présente un schéma unique ne permet pas de surveiller en continu la formation des dépôts et/ou le suivi de la stabilité thermique et la stabilité à l'oxydation du carburant.

La demande de brevet IN02374DE2013 A concerne l'utilisation de la micro-fluidique pour estimer la présence de contaminants dans les produits pétroliers en se basant sur le suivi de la conductivité comme mode de détection. Toutefois, ce système ne permet pas de surveiller en continu la formation des dépôts et/ou le suivi de la stabilité thermique et la stabilité à l'oxydation du carburant.

La demande de brevet FR3071062 A1 (WO2019052826 A1) concerne un dispositif de mesure de la stabilité à l'oxydation et/ou de la stabilité thermique de tout type de carburant, y compris le diesel, par une miniaturisation du système de test grâce à la technique micro-fluidique. Les phénomènes physiques auxquels sont soumis les carburants sont reproduits par les micro-canaux de la puce micro-fluidique, qui comportent des moyens représentatifs de l'injection du carburant et/ou la circulation du carburant pour un système d'entraînement, par exemple un moteur à combustion interne, ou un réacteur aéronautique. Ce type de dispositif donne satisfaction en termes de mesure de stabilité à l'oxydation et/ou de la stabilité thermique. Néanmoins, il est souhaitable d'améliorer la précision et la fiabilité des mesures, et de pouvoir quantifier les dépôts.

Le document : Michal Borecki et al. : « Capillary sensor with UV-VIS reading of effects of diesel and biodiesel fuel dégradation in storage », Sensors & Transducers, 1 octobre 2016, page 1-9, XP055474884 décrit la mesure de la stabilité de l'oxydation d'un diesel par fluorescence.

La demande de brevet US2007187617 A1 décrit une méthode et un dispositif de surveillance de l'oxydation d'une huile.

### Résumé de l'invention

La présente invention a pour objectif de mesurer, de manière fiable et précise, la stabilité à l'oxydation et/ou la stabilité thermique d'un carburant. L'invention concerne un dispositif et une méthode de mesure de la stabilité à l'oxydation et/ou de la stabilité thermique d'un carburant, au moyen d'une puce micro-fluidique ou milli-fluidique, et des moyens de mesure couplés de fluorescence et de variation de la capacité thermique du carburant. Les mesures couplées permettent de déterminer précisément et de manière fiable la stabilité thermique et/ou la et/ou la stabilité à l'oxydation du carburant. L'invention permet également de détecter la formation de dépôt (liquide ou solide) au sein du carburant.

L'invention concerne un dispositif de mesure de la stabilité à l'oxydation et/ou de la stabilité thermique d'un carburant, selon la revendication 1.

Selon un mode de réalisation, lesdits moyens de mesure de la variation de la capacité thermique dudit carburant comprennent un débitmètre thermique agencé sur un canal de ladite puce micro-fluidique ou milli-fluidique.

Conformément à un mode de réalisation, lesdits moyens de mesure de la variation de la capacité thermique dudit carburant sont agencés en amont ou en aval desdits moyens de mesure de la fluorescence.

Avantageusement, lesdits moyens de mesure de la fluorescence dudit carburant sont orientés vers un canal de ladite puce micro-fluidique ou milli-fluidique.

Selon un aspect, lesdits moyens de mesure de la fluorescence dudit carburant comprennent une source d'émission de lumière, notamment une source monochromatique, de préférence une source LASER ou LED, et un récepteur optique, notamment une photodiode, un photomultiplicateur ou une photodiode avalanche.

De préférence, des moyens de focalisation de la lumière sont agencés entre ladite source lumineuse et ladite puce micro-fluidique ou milli-fluidique, de préférence les moyens de focalisation de la lumière comprennent au moins une fibre multimodale et/ou un collimateur et/ou un filtre passe haut et/ou un filtre passe-bande.

De manière avantageuse, ladite source d'émission de lumière est agencée sensiblement perpendiculairement audit détecteur optique.

Conformément à une mise en oeuvre, ladite puce micro-fluidique ou milli-fluidique est en verre, silice, quartz, métal, polymère tel que le PDMS (PolyDiMethylSiloxane), les résines photosensibles, les polymères thermoplastiques, les colles.

Avantageusement, ledit carburant est un carburant de type carburéacteur, diesel, biodiesel, biocarburant, carburant alternatif, coupes de raffinage, essence, huiles, lubrifiants ou hydrocarbures.

Selon un mode de réalisation, lesdits moyens de circulation dudit carburant comprennent un pousse-seringue, un contrôleur de pression, ou une pompe péristaltique.

Selon une caractéristique, ledit dispositif comprend un moyen d'acquisition desdites mesures de variation de la capacité thermique dudit carburant et desdites mesures de la fluorescence dudit carburant.

En outre, l'invention concerne une méthode de mesure de la stabilité à l'oxydation et/ou de la stabilité thermique d'un carburant, selon la revendication 11.

Selon un mode de réalisation, on corrèle ladite mesure de la variation de la capacité thermique dudit carburant et ladite mesure de fluorescence pour déterminer et/ou quantifier la présence de dépôt dans ledit carburant.

Conformément à une mise en oeuvre, on réalise lesdites mesures en continu.

D'autres caractéristiques et avantages du dispositif et de la méthode selon l'invention, apparaîtront à la lecture de la description ci-après d'exemples non limitatifs de réalisations, en se référant aux figures annexées et décrites ci-après.

### Liste des figures

La figure 1 illustre un dispositif de mesure selon un premier mode de réalisation de l'invention.
La figure 2 illustre une vue en coupe des moyens de mesure de fluorescence selon un mode de réalisation de l'invention.
La figure 3 illustre un dispositif de mesure selon un deuxième mode de réalisation de l'invention.
La figure 4 illustre, pour un exemple, des courbes de l'intensité de fluorescence pour différents carburants acquises avec le dispositif selon un mode de réalisation de l'invention.
La figure 5 illustre, pour un exemple, un histogramme de la capacité thermique de différents carburants.
La figure 6 illustre, pour un exemple, des mesures au moyen d'un dispositif selon un mode de réalisation.

### Description des modes de réalisation

La présente invention concerne un dispositif de mesure de la stabilité à l'oxydation et/ou de la stabilité thermique d'un carburant. Le dispositif selon l'invention permet notamment de mesurer les dépôts, en d'autres termes l'encrassement, issus d'un carburant dans des conditions spécifiques, qui correspondent aux conditions d'utilisation du carburant. Ainsi, le dispositif selon l'invention peut être utilisé pour analyser les problèmes de colmatage et de formation de dépôts de type vernis, gomme, laque et coke dans des conditions d'utilisation.

On appelle stabilité à l'oxydation ou autoxydation la tendance d'un carburant à se dégrader à partir de l'oxydation de ses composés par le contact avec l'oxygène.

On appelle stabilité thermique ou thermo-oxydation la caractéristique d'un carburant à se dégrader ou à se décomposer ou a changé de composition à partir de l'exposition à des températures élevées dans un milieu avec ou sans oxygène. On peut noter que la température a un effet direct sur la cinétique des réactions de décomposition d'un carburant.

Les processus de thermo oxydation génèrent la formation des nouveaux composés chimiques. Les composés chimiques formés plus denses deviendront éventuellement insolubles jusqu'à la séparation d'une phase sédimentée, appelée dépôt (liquide ou solide) et d'une phase légère liquide appelée surnageante.

Dans la présente demande, le terme carburant désigne tous les hydrocarbures, les fluides complexes, les coupes de raffinage, les huiles ou lubrifiants. En particulier, le carburant testé peut être de tout type, en particulier un diesel, un biodiesel, une essence, un biocarburant, un carburant alternatif ou un carburéacteur. Selon un exemple, le carburant peut être un fluide de refroidissement, qui peut être sensible à une oxydation.

Le système selon l'invention comporte au moins :
- des moyens d'alimentation en carburant à tester, il peut s'agir notamment d'une réserve de carburant, ou de moyens de connexion à un système d'alimentation en carburant,
- une puce micro-fluidique ou milli-fluidique, qui comprend au moins un micro-canal ou respectivement milli-canal pour l'écoulement du carburant, et au sein de laquelle le carburant est dégradé et/ou forme des dépôts,
- des moyens de circulation du carburant au sein de la puce micro-fluidique ou milli-fluidique depuis les moyens d'alimentation, et
- des moyens de mesure liés à la puce micro-fluidique ou milli-fluidique, les moyens de mesures sont aptes à mesurer au moins une caractéristique du carburant circulant dans la puce micro-fluidique, et/ou une caractéristique liée au dépôt (encrassement) dans la puce micro-fluidique.

Une puce micro-fluidique (respectivement milli-fluidique) est un ensemble de micro-canaux (respectivement milli-canaux) gravés ou moulés dans un matériau (par exemple verre (allant du quartz à la silice fondue en passant par les sodocalciques et borosilicates), silicium, métal ou polymère tel que le PDMS, pour PolyDiMethylSiloxane, ou les résines photosensibles comme la SU-8, ou les polymères thermoplastiques, comme le PMMA ou le PEEK, ou colle tel que la NOA (Norland Optical Adhesive)). Classiquement, les micro-canaux (respectivement milli-canaux) constituant la puce micro-fluidique (respectivement milli-fluidique) sont connectés entre eux de manière à réaliser une fonction voulue (mélanges, pompage, tri, contrôle de l'environnement bio-chimique, mesures, analyses chimiques). Ce réseau de micro-canaux (respectivement milli-canaux) enfermés dans la puce micro-fluidique (respectivement milli-fluidique) est relié à l'extérieur par au moins une entrée et au moins une sortie percées à travers la puce, comme des interfaces entre le monde macroscopique et microscopique. Ce sont par ces trous que le carburant est injecté et évacué de la puce micro-fluidique (respectivement milli-fluidique) (au travers de tubes, adaptateurs à seringue ou même de simples trous dans la puce) avec des systèmes actifs extérieurs (contrôleur de pression, pousse-seringue ou pompes péristaltiques) ou des moyens passifs (par exemple des pressions hydrostatiques). Une puce micro-fluidique (respectivement milli-fluidique) peut fonctionner sous pression.

Selon l'invention, les moyens de mesure de la stabilité à l'oxydation et/ou de la stabilité thermique du carburant comprennent :
- des moyens de mesure de la variation de la capacité thermique du carburant, couplés avec
- des moyens de mesure de la fluorescence du carburant.

La mesure de la variation de la capacité thermique est basée sur la variation de propriétés thermiques entre la phase surnageante du carburant et son éventuel dépôt liquide/solide formé lors d'un processus avancé d'oxydation. En effet, les dépôts liquides formés présentent une capacité calorifique plus élevée que la capacité calorifique de la phase surnageante du carburant. Selon un mode de réalisation de l'invention, les moyens de mesure de la variation de la capacité thermique peuvent comprendre au moins un débitmètre thermique. De manière avantageuse, le débitmètre thermique peut être agencé sur un canal de la puce micro-fluidique ou milli-fluidique. En effet, l'augmentation de capacité calorifique du carburant se traduit par une augmentation du débit mesuré. Le principe de fonctionnement d'un débitmètre thermique est basé sur l'échauffement du fluide traversant le débitmètre. Par exemple, il peut comprendre deux sondes de température placées de part et d'autre d'une source de chaleur. La puissance thermique peut être fixe ou variable de manière à maintenir un différentiel de température constant entre les deux sondes. Le débit mesuré est alors déterminé en fonction de la vitesse de propagation de la chaleur jusqu'aux sondes thermiques. Alternativement, des calorimètres peuvent être utilisés pour mesurer la variation de capacité thermique.

Lors de l'oxydation d'un carburant, de nombreuses espèces chimiques sont formées, comme par exemple des cétones, des acides carboxyliques ou encore des esters. Même si les composés chimiques formés ne sont pas tous identifiés, la réaction du dioxygène avec les carburants va conduire à la formation de composés présentant une réponse en fluorescence. Les produits d'oxydation de différents carburants émettent un signal de fluorescence. Suivre la formation de ces nouvelles espèces chimiques à travers leur signal en fluorescence permet de connaitre l'avancée de l'oxydation du carburant.

Le couplage de ces deux mesures permet une mesure en continu précise et fiable. Ainsi, l'invention permet de quantifier le degré d'oxydation du carburant en suivant la formation des produits d'oxydation comme traceurs. En effet, sans la mesure de variation de la capacité thermique du carburant, il n'est pas possible de confirmer la présence de dépôt par le seul fait d'une augmentation du signal de fluorescence. Cela provient du fait que certains dépôts liquides formés dans le carburant pourraient ne pas présenter de différence d'intensité de fluorescence émise par une phase surnageante. Cette présence peut être identifiée uniquement par la mesure de variation de capacité thermique. De plus, le dispositif de mesure est adapté à tous types de carburant.

De préférence, les mesures de variation de la capacité thermique et de la fluorescence émise sont réalisées de manière simultanée, pour favoriser le couplage des mesures, et ainsi déterminer de manière fiable et précise la stabilité à l'oxydation et la stabilité thermique du carburant.

Selon un mode de réalisation de l'invention, les moyens de mesure de la variation de la capacité thermique peuvent être agencés en amont ou en aval des moyens de mesure de fluorescence, dans le sens de circulation du carburant dans le dispositif.

De préférence, les deux moyens de mesure peuvent être agencés sur un unique canal de la puce micro-fluidique ou milli-fluidique. De manière avantageuse, afin de favoriser les mesures et pour leur corrélation, le canal de la puce micro-fluidique ou milli-fluidique peut être un canal rectiligne (c'est-à-dire sans courbure, dérivation, etc.) pour les deux mesures et entre les deux emplacements des moyens de mesure.

De manière avantageuse, le canal de la puce micro-fluidique ou milli-fluidique peut avoir une section constante dans la zone des mesures, afin de ne pas perturber la circulation du carburant dans la puce micro-fluidique ou milli-fluidique.

Selon un aspect, le canal de la puce micro-fluidique ou milli-fluidique peut avoir toute forme adaptée aux deux moyens de mesure, par exemple circulaire, rectangulaire, carrée, etc. De préférence, la section du canal peut être sensiblement rectangulaire ou carrée de manière à faciliter les mesures de fluorescence.

Conformément à une mise en oeuvre de l'invention, les moyens de mesure de la fluorescence du carburant peuvent être orientés vers un canal de la puce micro-fluidique ou milli-fluidique.

Selon un aspect de l'invention, les moyens de mesure de la fluorescence du carburant peuvent comprendre une source d'émission de lumière et un récepteur optique, apte à capter la fluorescence émise du carburant générée par la source d'émission de lumière. De préférence, la source d'émission de lumière peut être monochromatique, par exemple au moyen d'un LASER ou d'une LED. Ainsi, une longueur d'onde spécifique a été sélectionnée afin de cibler les composés d'intérêt, et de s'affranchir du signal de fluorescence du carburant « pur ». Alternativement, la source d'émission de lumière peut prendre toute autre forme. De manière préférée, le récepteur optique peut être une photodiode, un photomultiplicateur ou une photodiode avalanche (APD de l'anglais « avalanche photodiode »). En variante, le récepteur optique peut prendre tout autre forme.

Pour ce mode de réalisation, le dispositif de mesure peut comprendre des moyens de focalisation de la lumière afin d'améliorer la sensibilité de la mesure de la fluorescence émise par le carburant. Les moyens de focalisation de la lumière peuvent comprendre au moins un élément choisi parmi une fibre multimodale, un collimateur, un filtre passe haut, un filtre passe-bande.

Afin de favoriser la mesure de la fluorescence, la source d'émission de la lumière peut être agencée perpendiculairement au détecteur optique.

La puce micro-fluidique ou milli-fluidique présente l'avantage d'avoir des dimensions réduites, notamment par rapport aux systèmes de l'art antérieur.

La plus petite dimension (hauteur ou largeur ou diamètre) des micro-canaux ou des milli-canaux est comprise entre quelques micromètres et quelques millimètres. En outre, les micro-canaux ou milli-canaux peuvent avoir des longueurs comprises entre quelques centimètres et quelques mètres.

Selon une mise en oeuvre de l'invention, le système micro-fluidique ou milli-fluidique peut être réalisé en verre, en silice ou en quartz, ou tout autre matériau compatible avec les carburants et les conditions d'utilisation. De plus, ces matériaux permettent de faciliter les mesures, en particulier les mesures de fluorescence.

Selon un mode de réalisation de l'invention, les moyens de circulation du fluide dans la puce micro-fluidique ou milli-fluidique peuvent être aptes à réguler le débit de carburant circulant dans la puce micro-fluidique ou milli-fluidique.

Afin de réguler le débit de circulation du fluide dans la puce micro-fluidique, les moyens de circulation de carburant peuvent être un pousse-seringue (avec un volume compris entre 10 µL et 50 mL), qui est adapté aux faibles volumes et débits mis en oeuvre par le dispositif de mesure selon l'invention.

En variante, les moyens de circulation de carburant peuvent être une pompe, par exemple une pompe péristaltique, ou une pompe à pression contrôlée (type « flow control system » de Fluigent).

Avantageusement, le dispositif de mesure peut comprendre en outre au moins l'un des équipements suivants :
- un réservoir d'échantillon du carburant à tester,
- un système d'oxydation in situ avec une variation de température et de pression
- des moyens d'automatisation de la mesure pour contrôler notamment les moyens de circulation du carburant et les moyens de mesure,
- un circuit de flush pour nettoyer le dispositif entre deux mesures, et
- un module d'analyse permettant de regrouper les mesures, etc.

De plus, le dispositif de mesure peut comprendre un moyen d'acquisition des mesures de variation de la capacité thermique du carburant et des mesures de la fluorescence du carburant. Les moyens d'acquisition sont configurés pour coupler (c'est-à-dire corréler) les mesures issues des deux moyens de mesure pour déterminer la stabilité à l'oxydation et la stabilité thermique, voire quantifier le dépôt au sein du carburant. Les moyens d'acquisition peuvent comprendre un système informatique.

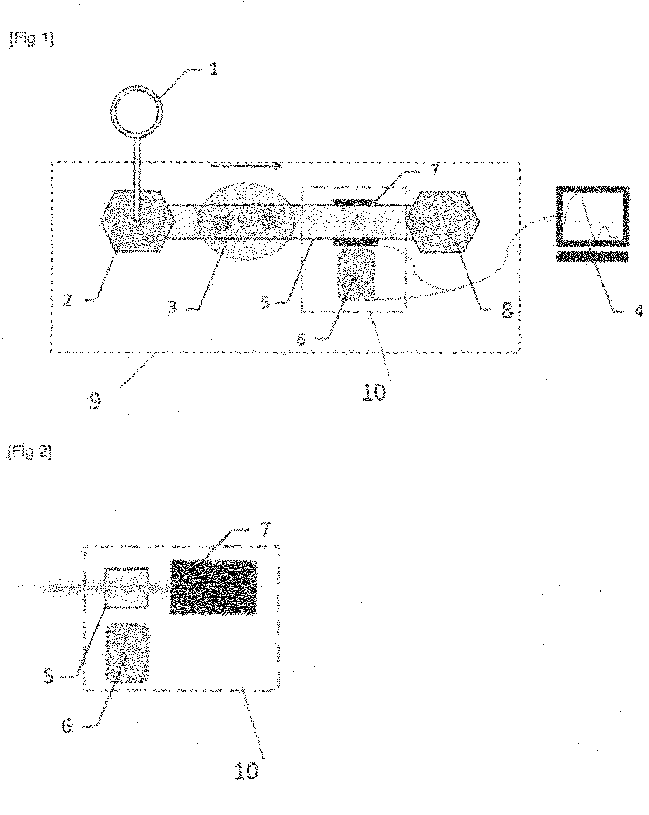

La figure 1 illustre, schématiquement et de manière non limitative, un dispositif de mesure selon un premier mode de réalisation de l'invention. Le dispositif de mesure comporte une source de pression 1 qui permet au carburant de circuler à la pression constante dans la puce micro-fluidique ou milli-fluidique 9. La circulation du carburant dans la puce micro-fluidique ou milli-fluidique 9 est illustrée par une flèche. La puce micro-fluidique ou milli-fluidique 9 comprend un réservoir d'entrée 2 et un réservoir de sortie 8. Les réservoirs d'entrée 2 et de sortie 8 sont reliés par un unique canal 5. Le canal 5 est un canal rectiligne entre le réservoir d'entrée 2 et le réservoir de sortie 8. Le canal 5 peut avoir une section sensiblement carrée. Un débitmètre thermique 3 est placé sur le canal 5, ainsi que des moyens de mesure de fluorescence 10. Pour le mode de réalisation de la figure 1, le débitmètre thermique 3 est agencé en amont des moyens de mesure de la fluorescence 10. Les moyens de mesure de la fluorescence 10 comprennent une source d'émission lumineuse 7, notamment un LASER ou une LED, dont le faisceau lumineux est orienté sensiblement perpendiculairement au plan de la figure 1. En outre, les moyens de mesure de la fluorescence 10 comprennent un récepteur optique 6, par exemple une photodiode avalanche. Pour le mode de réalisation illustré, le récepteur optique 6 est agencé perpendiculairement à la source d'émission lumineuse 7. De plus, le dispositif de mesure comprend des moyens d'acquisition 4 qui sont reliés notamment aux moyens de mesure de la fluorescence 10.

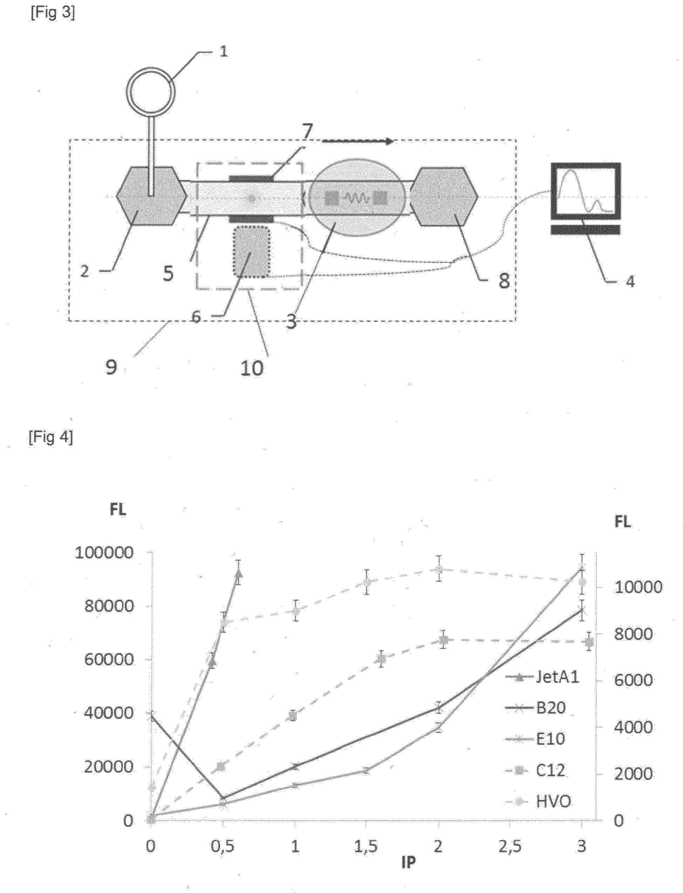

La figure 2 illustre, schématiquement et de manière non limitative, des moyens de mesure de la fluorescence 10 selon un mode de réalisation de l'invention. La figure 2 est une vue dans un plan de la section du canal 5 de la puce micro-fluidique ou milli-fluidique (vue perpendiculaire au plan de la figure 1). La source d'émission lumineuse 7 est agencée sur un côté du canal 5 pour émettre un faisceau lumineux qui traverse le canal 5. Le récepteur optique 6 est agencé sur un côté du canal 5 perpendiculaire du faisceau lumineux issue de la source d'émission lumineuse 7 de manière à analyser la fluorescence émise par le carburant en présence du faisceau lumineux. Les moyens de mesure de la fluorescence 10 de la figure 2 sont adaptés au dispositif de mesure de la figure 1, ainsi que pour le dispositif de mesure de la figure 3 qui sera détaillé dans la suite de la description.

La figure 3 illustre, schématiquement et de manière non limitative, un dispositif de mesure selon un deuxième mode de réalisation de l'invention, dans le même plan que la figure 1. Le dispositif de mesure comporte une source de pression 1 qui permet au carburant de circuler à un débit imposé dans la puce micro-fluidique ou milli-fluidique 9. La circulation du carburant dans la puce micro-fluidique ou milli-fluidique 9 est illustrée par une flèche. La puce micro-fluidique ou milli-fluidique 9 comprend un réservoir d'entrée 2 et un réservoir de sortie 8. Les réservoirs d'entrée 2 et de sortie 8 sont reliés par un unique canal 5. Le canal 5 est un canal rectiligne de section constante entre le réservoir d'entrée 2 et le réservoir de sortie 8. Le canal 5 peut avoir une section sensiblement carrée. Un débitmètre thermique 3 est placé sur le canal 5, ainsi que des moyens de mesure de fluorescence 10. Pour le deuxième mode de réalisation de la figure 3, le débitmètre thermique 3 est agencé en aval des moyens de mesure de la fluorescence 10. Les moyens de mesure de la fluorescence 10 comprennent une source d'émission lumineuse 7, notamment un LASER ou une LED, dont le faisceau lumineux est orienté sensiblement perpendiculairement au plan de la figure 3. En outre, les moyens de mesure de la fluorescence 10 comprennent un récepteur optique 6, par exemple une photodiode avalanche. Pour le mode de réalisation illustré, le récepteur optique 6 est agencé perpendiculairement à la source d'émission lumineuse 7. De plus, le dispositif de mesure comprend des moyens d'acquisition 4 qui sont reliés notamment aux moyens de mesure de la fluorescence 10.

En outre, l'invention concerne une méthode de mesure de la stabilité à l'oxydation et/ou de la stabilité thermique d'un carburant au moyen d'un dispositif de mesure selon une variante ou l'une des combinaisons de variantes décrites ci-dessus. Selon l'invention, on réalise une mesure de la variation de la capacité thermique du carburant et une mesure de la fluorescence émise du carburant.

Selon un mode de réalisation de l'invention, on peut corréler (coupler) la mesure de la variation de la capacité thermique du carburant et la mesure de la fluorescence du carburant pour déterminer et/ou quantifier la présence de dépôt dans le carburant. En d'autres termes, on associe les mesures issues des deux technologies (fluorescence et capacité thermique) pour déterminer et/ou quantifier le dépôt.

Afin de prendre en compte le temps de déplacement du carburant dans le canal de la puce micro-fluidique ou milli-fluidique, on peut réaliser un décalage temporaire de la mesure qui est réalisée en premier sur le canal (en amont dans le sens de circulation du carburant). Le décalage temporaire peut être déterminé en fonction du débit du carburant dans la puce micro-fluidique ou milli-fluidique.

De manière avantageuse, les deux mesures peuvent être réalisées en continu de manière à suivre dans le temps la stabilité du carburant, et la formation des dépôts.

Pour la méthode, on peut utiliser une référence qui le plus souvent sera le carburant non-oxydé ou un modèle carburant (ou le partie surnageante du carburant dans le cas où l'on veut détecter la formation de dépôt).

De préférence, on peut réaliser les mesures de variation de la capacité thermique et de la fluorescence de manière simultanée, pour favoriser le couplage des mesures, et ainsi déterminer de manière fiable et précise la stabilité à l'oxydation et la stabilité thermique du carburant.

Comme il va de soi, l'invention ne se limite pas aux seules formes de réalisation du dispositif et de la méthode de mesure, décrits ci-dessus à titre d'exemple, elle embrasse au contraire toutes les variantes de réalisation dans le cadre des revendications annexées.

### Exemples

Les caractéristiques et avantages du procédé selon l'invention apparaîtront plus clairement à la lecture des exemples d'application ci-après.

### Premier exemple :

Pour le premier exemple, le dispositif de mesure selon l'invention est testé en utilisant différents types de carburants à différents niveaux d'oxydation pour montrer que le dispositif de mesure selon l'invention a une sensibilité assez grande pour détecter une variation dans la composition chimique des carburants durant leur oxydation.

Les carburants ou les modèles de carburants utilisés ont subi une oxydation thermique accélérée et contrôlée au sein d'un autoclave. 50 mL de carburant sont introduits dans un autoclave de volume total de 250 mL. L'enceinte est ensuite remplie d'un gaz inerte, tel que l'hélium, avant d'être portée à la température de 130°C. Une fois la température au sein du carburant stable, le gaz inerte est remplacé par du dioxygène jusqu'à atteindre une pression avoisinant les 10 bars. On définit l'oxydation d'un carburant en parlant de période d'induction (IP) qui correspond à la diminution du pourcentage de gaz présent dans l'enceinte de l'autoclave. Une IP égale à 1 correspond à la diminution de 10 % de la pression totale de gaz.

Le montage utilisé présente les caractéristiques suivantes :
- Source de pression Fluigent MFCS^{™}-EZ: 2 bars
- Réservoirs entrée/sortie de la puce milli-fluidique de 15 mL
- Débitmètre(s) thermique(s) : Fluigent FLOW UNITS de taille S (150 µm de diamètre interne) et M (430 µm de diamètre interne)
- Carte d'acquisition NI USB-6356
- Canal millifluidique de section carrée en verre (700 µm de hauteur interne)
- Capteur optique : APD
- Source d'émission lumineuse : LASER de longueur d'onde de 488 nm avec une tension de 800 mV

Des filtres passe-haut à 500 nm permettent de s'affranchir de la longueur d'onde d'excitation 488 nm. Ce qui signifie que le signal collecté par la photodiode avalanche est uniquement le résultat de la fluorescence émise par les carburants.

Les courbes de la figure 4 illustrent la fluorescence mesurée FL au moyen du dispositif de mesure en fonction de la période d'induction IP pour différents carburants : un Jet A1 de référence (JetA1, type kérosène), un mélange en masse de 20 % de B100 RME (biodiesel) avec 80 % de B0 (pétrodiesel) (B20, type diesel), une essence Euro6 E10 (E10, type essence), du n-dodécane 99% pur de Sigma-Aldrich (C12, type hydrocarbure de référence) et une huile végétale hydrotraitée (HVO, type biodiesel). Sur cette figure, l'axe des ordonnées à gauche correspond aux courbes en trait continu (JetA1, B20, E10), et l'axe des ordonnées à droite correspond aux courbes en traits pointillés (C12, HVO). La figure met en évidence la variation de l'intensité de fluorescence émise à la longueur d'onde d'excitation de 488 nm, en fonction de la période d'induction IP du carburant étudié. De manière générale, on remarque une augmentation de l'intensité de fluorescence émise avec le temps d'exposition du carburant au dioxygène à 130°C, aut rement dit en fonction de l'IP, et cela pour tous les types de carburants. Le dispositif selon l'invention permet donc de quantifier le degré d'oxydation des carburants en suivant la formation des produits d'oxydation comme traceurs pour différents types de carburants.

### Deuxième exemple :

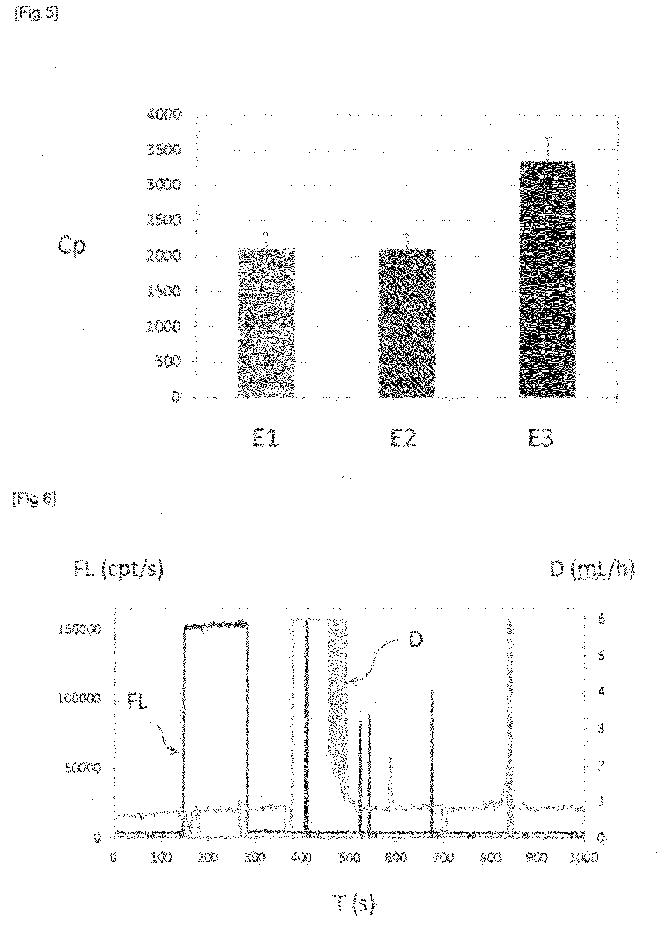

Un calorimètre différentiel à balayage (DSC) METTLER TOLEDO a été utilisé pour mesurer exsitu la capacité calorifique massique du n-dodécane pur (appelé échantillon E1) du n-dodécane oxydé à 1 IP (ou phase surnageante et appelé échantillon E2) et du dépôt liquide formé à 1 IP en autoclave (appelé échantillon E3). Les résultats sont présentés sur la Figure 5. La capacité calorifique Cp du dépôt liquide (E3) augmente fortement comparativement à celle du n-dodécane pur oxydé (E2). Les débitmètres thermiques permettent donc bien de déterminer la présence de dépôt au sein de la phase surnageante du dodécane, cela se traduit par une augmentation de la capacité calorifique massique, et donc du débit mesuré.

### Troisième exemple :

Le graphique présenté en figure 6 est un résultat type obtenu avec le dispositif de l'invention lors de la circulation de carburant avec dépôts liquides. Dans la figure 6, la courbe gris clair représente le signal d'un débitmètre thermique D en mL/h en fonction du temps T en s, et la courbe en gris foncé est la mesure de fluorescence FL en compte/seconde collecté par l'APD. Le débit mesuré avec un débitmètre Fluigent S est situé en aval du capillaire de section carrée (mode de réalisation de la figure 3). Lorsque le signal augmente fortement jusqu'à la saturation du débitmètre thermique (> 6 mL.h-1), cela traduit la présence de dépôt due à une forte variation de la capacité calorifique massique du dépôt. On remarque une forme similaire des deux courbes avec un décalage dans le temps de l'une par rapport à l'autre. Par exemple, l'augmentation du signal de fluorescence (au moyen d'un APD collectant les photons émis par fluorescence) à partir de 150 s correspond au dépôt analysé dans le débitmètre autour de 380 s d'expérience (le décalage de 230 s correspond au temps de circulation du carburant dans la puce micro-fluidique ou milli-fluidique). Lorsque le signal du débitmètre est nul cela correspond à la présence de bulles dans le débitmètre. La corrélation des deux mesures (après décalage temporaire de la courbe FL de 230s) permet de mesurer la stabilité à l'oxydation et/ou la stabilité thermique d'un carburant et de déterminer la formation des dépôts. Sans l'information D issue du débitmètre on ne peut pas confirmer la présence de dépôt par le seul fait d'une augmentation signification de signal de fluorescence FL. En effet, certains dépôts liquides formés dans des carburants pourraient ne pas présenter de différence d'intensité de fluorescence émise avec la phase surnageante. Leur présence est identifiée uniquement par le débitmètre thermique. Cet exemple montre la nécessité du couplage des deux techniques pour les objectifs visés par cette invention.

## Revendications

1. Dispositif de mesure de la stabilité à l'oxydation et/ou de la stabilité thermique d'un carburant, ledit système de mesure comprenant des moyens d'alimentation en carburant (1), une puce micro-fluidique ou milli-fluidique (9), qui comprend au moins un canal (5) pour l'écoulement du carburant, des moyens de circulation dudit carburant au sein de ladite puce micro-fluidique ou milli-fluidique, et des moyens de mesure de la stabilité à l'oxydation et/ou de la stabilité thermique dudit carburant, **caractérisé en ce que** lesdits moyens de mesure de la stabilité à l'oxydation et/ou de la stabilité thermique dudit carburant comprennent des moyens de mesure couplés, liés à ladite puce, et aptes à mesurer la variation de la capacité thermique dudit carburant (3) et la fluorescence dudit carburant (10).

2. Dispositif de mesure selon la revendication 1, dans lequel lesdits moyens de mesure aptes à mesurer la variation de la capacité thermique dudit carburant comprennent un débitmètre thermique (3) agencé sur le canal (5) de ladite puce micro-fluidique ou milli-fluidique (9).

3. Dispositif de mesure selon l'une des revendications précédentes, dans lequel lesdits moyens de mesure aptes à mesurer la variation de la capacité thermique (3) dudit carburant sont agencés en amont ou en aval desdits moyens de mesure de la fluorescence (10).

4. Dispositif de mesure selon l'une des revendications précédentes, dans lequel lesdits moyens de mesure aptes à mesurer la fluorescence (10) dudit carburant sont orientés vers le canal (5) de ladite puce micro-fluidique ou milli-fluidique (9).

5. Dispositif de mesure selon l'une des revendications précédentes, dans lequel lesdits moyens de mesure apte à mesurer la fluorescence (10) dudit carburant comprennent une source d'émission de lumière (7), notamment une source monochromatique, de préférence une source LASER ou LED, et un récepteur optique (6), notamment une photodiode, un photomultiplicateur ou une photodiode avalanche.

6. Dispositif de mesure selon la revendication 5, dans lequel des moyens de focalisation de la lumière sont agencés entre ladite source lumineuse (7) et ladite puce micro-fluidique ou milli-fluidique (9), de préférence les moyens de focalisation de la lumière comprennent au moins une fibre multimodale et/ou un collimateur et/ou un filtre passe haut et/ou un filtre passe-bande.

7. Dispositif de mesure selon l'une des revendications 5 ou 6, dans lequel ladite source d'émission de lumière (7) est agencée sensiblement perpendiculairement audit détecteur optique (6).

8. Dispositif de mesure selon l'une des revendications précédentes, dans lequel ladite puce micro-fluidique ou milli-fluidique (9) est en verre, silice, quartz, métal, polymère tel que le PDMS (PolyDiMethylSiloxane), les résines photosensibles, les polymères thermoplastiques, les colles.

9. Dispositif de mesure selon l'une des revendications précédentes, dans lequel lesdits moyens de circulation dudit carburant comprennent un pousse-seringue, un contrôleur de pression, ou une pompe péristaltique.

10. Dispositif de mesure selon l'une des revendications précédentes, dans lequel ledit dispositif comprend un moyen d'acquisition (4) desdites mesures de variation de la capacité thermique dudit carburant et desdites mesures de la fluorescence dudit carburant.

11. Méthode de mesure de la stabilité à l'oxydation et/ou de la stabilité thermique d'un carburant au moyen d'un dispositif de mesure selon l'une des revendications précédentes, **caractérisé en ce qu'**on réalise une mesure de la variation de la capacité thermique dudit carburant et une mesure de la fluorescence dudit carburant.

12. Méthode de mesure selon la revendication 11, dans lequel on corrèle ladite mesure de la variation de la capacité thermique dudit carburant et ladite mesure de fluorescence pour déterminer et/ou quantifier la présence de dépôt dans ledit carburant.

13. Méthode de mesure selon l'une des revendications 11 ou 12, dans lequel on réalise lesdites mesures en continu.

14. Méthode de mesure selon l'une des revendications 11 à 13, dans lequel ledit carburant est un carburant de type carburéacteur, diesel, biodiesel, biocarburant, carburant alternatif, coupes de raffinage, essence, huiles, lubrifiants ou hydrocarbures.

## Patentansprüche

1. Messvorrichtung zum Messen der Oxidationsstabilität und/oder der thermischen Stabilität eines Brennstoffs, wobei das Messsystem Brennstoffzuführungsmittel (1), einen mikrofluidischen oder millifluidischen Chip (9), der mindestens einen Kanal (5) zum Durchfließen des Brennstoffs umfasst, Zirkulationsmittel zur Zirkulation des Brennstoffs in dem mikrofluidischen oder millifluidischen Chip und Messmittel zum Messen der Oxidationsstabilität und/oder der thermischen Stabilität des Brennstoffs umfasst, **dadurch gekennzeichnet, dass** die Messmittel zum Messen der Oxidationsstabilität und/oder der thermischen Stabilität des Brennstoffs gekoppelte Messmittel umfassen, die mit dem Chip verbunden sind und geeignet sind, die Änderung der Wärmekapazität des Brennstoffs (3) und die Fluoreszenz des Brennstoffs (10) zu messen.

2. Messvorrichtung nach Anspruch 1, bei der die Messmittel, die geeignet sind, die Änderung der Wärmekapazität des Brennstoffs zu messen, einen thermischen Durchflussmesser (3) umfassen, der auf dem Kanal (5) des mikrofluidischen oder millifluidischen Chips (9) angeordnet ist.

3. Messvorrichtung nach einem der vorhergehenden Ansprüche, bei der die Messmittel, die geeignet sind, die Änderung der Wärmekapazität (3) des Brennstoffs zu messen, stromauf oder stromab der Messmittel zum Messen der Fluoreszenz (10) angeordnet sind.

4. Messvorrichtung nach einem der vorhergehenden Ansprüche, bei der die Messmittel, die geeignet sind, die Fluoreszenz (10) des Brennstoffs zu messen, zu dem Kanal (5) des mikrofluidischen oder millifluidischen Chips (9) hin ausgerichtet sind.

5. Messvorrichtung nach einem der vorhergehenden Ansprüche, bei der die Messmittel, die geeignet sind, die Fluoreszenz (10) des Brennstoffs zu messen, eine Lichtemissionsquelle (7), insbesondere eine monochromatische Quelle, bevorzugt eine LASER- oder LED-Quelle, und einen optischen Empfänger (6), insbesondere eine Photodiode, einen Photomultiplier oder eine Avalanche-Photodiode, umfassen.

6. Messvorrichtung nach Anspruch 5, bei der Mittel zum Fokussieren des Lichts zwischen der Lichtquelle (7) und dem mikrofluidischen oder millifluidischen Chip (9) angeordnet sind, die Mittel zum Fokussieren des Lichts bevorzugt mindestens eine multimodale Faser und/oder einen Kollimator und/oder ein Hochpassfilter und/oder ein Bandpassfilter umfassen.

7. Messvorrichtung nach einem der Ansprüche 5 oder 6, bei der die Lichtemissionsquelle (7) im Wesentlichen senkrecht zu dem optischen Detektor (6) angeordnet ist.

8. Messvorrichtung nach einem der vorhergehenden Ansprüche, bei welcher der mikrofluidische oder millifluidische Chip (9) aus Glas, Siliciumdioxid, Quarz, Metall, Polymer wie etwa PDMS (Polydimethylsiloxan), lichtempfindlichen Harzen, thermoplastischen Polymeren, Klebstoffen ist.

9. Messvorrichtung nach einem der vorhergehenden Ansprüche, bei der die Zirkulationsmittel zur Zirkulation des Brennstoffs eine Spritzenpumpe, einen Druckregler oder eine Schlauchpumpe umfassen.

10. Messvorrichtung nach einem der vorhergehenden Ansprüche, bei der die Vorrichtung ein Erfassungsmittel (4) zum Erfassen der Änderungsmessungen der Wärmekapazität des Brennstoffs und der Messungen der Fluoreszenz des Brennstoffs umfasst.

11. Messverfahren zum Messen der Oxidationsstabilität und/oder der thermischen Stabilität eines Brennstoffs mittels einer Messvorrichtung nach einem der vorhergehenden Ansprüche, **dadurch gekennzeichnet, dass** man eine Messung der Änderung der Wärmekapazität des Brennstoffs und eine Messung der Fluoreszenz des Kraftstoffs ausführt.

12. Messverfahren nach Anspruch 11, bei dem man die Messung der Änderung der Wärmekapazität des Brennstoffs und die Fluoreszenzmessung korreliert, um das Vorhandensein von Ablagerungen in dem Brennstoff zu bestimmen und/oder zu quantifizieren.

13. Messverfahren nach einem der Ansprüche 11 oder 12, bei dem man die Messungen kontinuierlich ausführt.

14. Messverfahren nach einem der Ansprüche 11 bis 13, bei dem der Brennstoff ein Brennstoff vom Typ Düsenkraftstoff, Diesel, Biodiesel, Biokraftstoff, alternativer Kraftstoff, Raffinationsfraktionen, Benzin, Öle, Schmierstoffe oder Kohlenwasserstoffe ist.

## Claims

1. Measurement device for measuring the oxidation stability and/or the thermal stability of a fuel, said measurement system comprising fuel supply means (1), a microfluidic or millifluidic chip (9) which comprises at least one channel (5) for the flow of the fuel, means for circulating said fuel in said microfluidic or millifluidic chip, and means for measuring the oxidation stability and/or the thermal stability of said fuel, **characterized in that** said means for measuring the oxidation stability and/or the thermal stability of said fuel comprise coupled measurement means connected to said chip and able to measure the variation in heat capacity of said fuel (3) and the fluorescence of said fuel (10).

2. Measurement device according to Claim 1, wherein said measurement means able to measure the variation in the heat capacity of said fuel comprise a thermal flow meter (3) arranged on the channel (5) of said microfluidic or millifluidic chip (9).

3. Measurement device according to one of the preceding claims, wherein said measurement means able to measure the variation in the heat capacity (3) of said fuel are arranged upstream or downstream of said means for measuring the fluorescence (10).

4. Measurement device according to one of the preceding claims, wherein said measurement means able to measure the fluorescence (10) of said fuel face toward the channel (5) of said microfluidic or millifluidic chip (9) .

5. Measurement device according to one of the preceding claims, wherein said measurement means able to measure the fluorescence (10) of said fuel comprise a light emitting source (7), notably a monochromatic source, preferably a laser or LED source, and an optical receiver (6), notably a photo diode, a photo multiplier or an avalanche photo diode.

6. Measurement device according to Claim 5, wherein means for focussing the light are arranged between said light source (7) and said microfluidic or millifluidic chip (9), the light focussing means preferably comprising at least one multimode fibre and/or a collimator and/or a high-pass filter and/or a bandpass filter.

7. Measurement device according to one of Claims 5 or 6, wherein said light emitting source (7) is arranged substantially perpendicular to said optical detector (6).

8. Measurement device according to one of the preceding claims, wherein said microfluidic or millifluidic chip (9) is made of glass, silica, quartz, metal, polymer such as PDMS (polydimethylsiloxane), photosensitive resins, thermoplastic polymers, adhesives.

9. Measurement device according to one of the preceding claims, wherein said means for circulating said fuel comprise a syringe driver, a pressure controller or a peristaltic pump.

10. Measurement device according to one of the preceding claims, wherein said device comprises an acquisition means (4) for acquiring said measurements of the variation in heat capacity of said fuel and said measurements of the fluorescence of said fuel.

11. Measurement method for measuring the oxidation stability and/or the thermal stability of a fuel by means of a measurement device according to one of the preceding claims, **characterized in that** a measurement of the variation in heat capacity of said fuel and a measurement of the fluorescence of said fuel are taken.

12. Measurement method according to Claim 11, wherein said measurement of the variation in heat capacity of said fuel and said fluorescence measurement are correlated in order to determine and/or quantify the presence of deposits in said fuel.

13. Measurement method according to one of Claims 11 or 12, wherein said measurements are taken continuously.

14. Measurement method according to one of Claims 11 to 13, wherein said fuel is a fuel of the aviation fuel, diesel, biodiesel, biofuel, alternative fuel, refinery cut, petroleum, oil, lubricant or hydrocarbon type.
